# EUROPÄISCHE PATENTSCHRIFT

(11) **EP 2 467 178 B1**
(45) Veröffentlichungstag und Bekanntmachung des Hinweises auf die Patenterteilung: **04.12.2013**
(21) Anmeldenummer: 10747821.6
(22) Anmeldetag: 13.08.2010
(51) Int. Cl.: A61M 1/34, A61M 1/36

(54) **SCHLAUCHSET FÜR EINE VORRICHTUNG ZUR BLUTBEHANDLUNG UND VORRICHTUNG ZUR BLUTBEHANDLUNG MIT EINEM SCHLAUCHSET**
TUBE SET FOR A BLOOD TREATMENT APPARATUS AND BLOOD TREATMENT APPARATUS COMPRISING A TUBE SET
ENSEMBLE DE CONDUITE FLEXIBLE POUR UN DISPOSITIF DE TRAITEMENT DU SANG, ET DISPOSITIF DE TRAITEMENT DU SANG PRÉSENTANT UN ENSEMBLE DE CONDUITE FLEXIBLE

(30) Priorität: 19.08.2009 DE 102009037917
(43) Veröffentlichungstag der Anmeldung: 27.06.2012
(73) Patentinhaber: Fresenius Medical Care Deutschland GmbH, 61352 Bad Homburg v.d.H. (DE)
(72) Erfinder: HERRENBAUER, Michael, 61267 Neu-Anspach (DE); POHLMEIER, Robert, 61350 Bad Homburg (DE); LAUBROCK, Andreas, 61273 Wehrheim (DE); BACHMANN, Angelika, 61191 Rosbach v.d.H. (DE)
(74) Vertreter: Oppermann, Frank
(86) Internationale Anmeldenummer: PCT/EP2010/004985
(87) Internationale Veröffentlichungsnummer: WO 2011/020585

(56) Entgegenhaltungen:
- WO-A2-02/35979
- US-A- 4 391 599
- US-A- 5 605 545

## Beschreibung

Die Erfindung betrifft ein Schlauchset für eine Vorrichtung zur Blutbehandlung, insbesondere eine Vorrichtung zur extrakorporalen Blutbehandlung mit einem extrakorporalen Blutkreislauf oder eine Vorrichtung zur Peritonealdialyse. Darüber hinaus betrifft die Erfindung eine Vorrichtung zur Blutbehandlung, insbesondere eine extrakorporale Blutbehandlungsvorrichtung oder eine Vorrichtung zur Peritonealdialyse, die über ein derartiges Schlauchset verfügt.

Es sind verschiedene Verfahren zur Blutbehandlung bekannt. Bei einer extrakorporalen Blutbehandlung wird das Blut des Patienten in einem extrakorporalen Blutkreislauf gereinigt, der eine Austauscheinheit, beispielsweise einen Dialysator oder Filter umfasst. Der Dialysator oder Filter weist eine Blutkammer und eine Dialysierflüssigkeitskammer auf, die von einer semipermeablen Membran getrennt sind. Bei der Hämodialyse im engeren Sinn wird die Blutkammer des Dialysators von Blut und die Dialysierflüssigkeitskammer von Dialysierflüssigkeit durchflossen. Bei der Hämofiltration wird die dem Patienten über die semipermeable Membran des Dialysators oder Filters entzogenen Flüssigkeit ganz oder teilweise durch eine sterile Substitutionsflüssigkeit (Substituat) ersetzt, wobei dies entweder stromauf oder stromab des Dialysators möglich ist. Hämodialyse und Hämofiltration können auch in einem Aufbau kombiniert werden, was als Hämodiafiltration bezeichnet wird. Diese Varianten werden im Folgenden als Hämodialyse im weiteren Sinne bezeichnet.

Neben der Hämodialyse ist auch die Peritonealdialyse bekannt, bei der über einen implantierten Peritonealkatheter eine Peritoneallösung dem Peritonealraum des Patienten zugeführt und aus dem Peritonealraum abgeführt wird. Bei der Peritonealdialyse erfolgt die Blutbehandlung durch den Peritonealraum hindurch.

Die Dialysierflüssigkeit kann bei der Hämodialyse mit einer zentralen Versorgungseinheit bereitgestellt werden. Das Substituat kann aus Frischwasser und Konzentrat oder aus der Dialysierflüssigkeit gewonnen werden. Bei der akuten Hämodialyse ist es bekannt, Dialysierflüssigkeit und Substituat zu verwenden, das in Beuteln bereitgestellt wird. Zur Bereitstellung von Dialysierflüssigkeit und Substituat sind auch Mehrkammerbeutel bekannt, die mit verschiedenen Komponenten zur Herstellung der Dialysierflüssigkeit oder des Substituats befüllt sind. Die einzelnen Komponenten werden üblicherweise erst kurz vor der Behandlung zur Herstellung der fertigen Flüssigkeit miteinander vermischt.

Bekannte Lösungsbeutel zur Herstellung von Dialysier- oder Substitutionsflüssigkeit weisen zwei getrennte Kammern auf, von denen die eine Kammer mit Bikarbonat als alkalische Lösung und die andere Kammer mit einer sauren Lösung aus Calcium und Elektrolyten befüllt sind. Die beiden Kammern des von einer Umverpackung umschlossenen Zweikammerbeutels sind von einer auftrennbaren Naht getrennt. Nach Entnehmen des Beutels aus der Umverpackung und Auftrennen der Naht vermengen sich beide Lösungen zu einer pH-Wert neutralen Dialysier- oder Substitutionsflüssigkeit mit einem in etwa physiologischen pH-Wert. Je nach Ausführungsform ist in der Primärverpackung und/oder der Umverpackung eine Gasbarriere integriert, die ein Ausgasen von CO₂ aus der Bikarbonatlösung und eine daraus folgende Verschiebung des pH-Wertes zu höheren Werten hin verhindert. Durch unsachgemäße Lagerung und Handhabung kann es aber zu einer Beschädigung der Verpackung kommen, so dass sich der pH-Wert der Bikarbonatlösung zu höheren Werten hin verschiebt. In einer solchen Lösung kann es zur Ausfällung von Calciumcarbonat in der fertigen Dialysier- oder Substitutionsflüssigkeit als fester Bestandteil kommen. Die Infusion dieser Partikel stellt dann eine Gefährdung für den Patienten dar.

Das Problem der Infusion ausfällender fester Bestandteile stellt sich auch bei der Peritonealdialyse, bei der die Peritoneallösung in einem Mehrkammerbeutel bereitgestellt wird, der die erst kurz vor der Behandlung miteinander zu vermischenden Komponenten enthält. Die Peritoneallösung unterscheidet sich von der Dialysierflüssigkeit im Wesentlichen durch den Glucose-Gehalt. Auch hier kann es bei unsachgemäßer Lagerung oder Beschädigung der Verpackung zum Ausfällen von Calciumcarbonat als feste Bestandteile kommen, die in den Peritonealraum des Patienten gelangen können. Auch können weitere nicht gelöste Stoffe oder Teile, beispielsweise Splitter von Brechkonen, in der Peritoneallösung enthalten sein.

Die bekannten Blutbehandlungsvorrichtungen verfügen über Schlauchleitungen, die zur einmaligen Verwendung bestimmt sind. Derartige Schlauchleitungen werden als Sets angeboten. Die Schlauchleitungen werden in okkludierende Pumpen, insbesondere Rollenpumpen, eingelegt. Unter okkludierenden Pumpen werden im Folgenden auch Pumpsysteme aus einer nicht okkludierenden Pumpe, beispielsweise einer Zentrifugalpumpe, mit vor- und/oder nachgeschalteten Ventilen verstanden, die von einer Steuerung derart angesteuert werden, dass sich das System aus Ventil oder Ventilen und nicht okkludierender Pumpe wie einer okkludierende Pumpe verhält.

Bei der Blutbehandlung sind zur Bilanzierung von frischer gegen verbrauchte Dialysierflüssigkeit Bilanziereinrichtungen bekannt. Es sind Bilanziereinrichtungen bekannt, bei denen die Beutel, in denen die frische Dialysierflüssigkeit bereitgestellt und die verbrauchte Dialysierflüssigkeit aufgenommen werden, gewogen werden. Daher verfügen diese Bilanziereinrichtungen über Waagen für die Lösungsbeutel.

Die WO 02/35979 A2 beschreibt eine Blutbehandlungsvorrichtung, die einen extrakorporalen Blutkreislauf aufweist, der eine arterielle Schlauchleitung und eine venöse Schlauchleitung umfasst, wobei die arterielle Leitung Blut zu der Blutkammer des Blutfilters führt und die venöse Leitung Blut aus der Blutkammer abführt. Dabei wird die Blutkammer von Blut durchströmt. Über die Membran des Filters wird dem extrakorporalen Blutkreislauf Ultrafiltrat entzogen. Das Blut wird mit einer okkludierenden Schlauchpumpe gefördert, die an der arteriellen Leitung vorgesehen ist. Die Schlauchpumpe weist federnd vorgespannte Rollen auf, mit denen die Schlauchleitung okkludiert wird. Die Vorspannung ist dabei so eingestellt, dass bei einem bestimmten Druck im extrakorporalen Blutkreislauf die Rollen von der Schlauchleitung abheben, so dass die Schlauchleitung nicht mehr vollständig okkludiert ist. Dadurch wird vermieden, dass sich der Druck im extrakorporalen Blutkreislauf so stark erhöhen kann, dass die Membran des Filters beschädigt werden könnte.

Die US-A-5,605,545 beschreibt eine medizinische Anordnung mit Beuteln zur Aufnahme von Flüssigkeiten, an denen eine Schlauchleitung angeschlossen ist, die zu einem chirurgischen Instrument führt. Die Flüssigkeiten aus den Beuteln werden mit einer okkludierenden Schlauchpumpe gefördert. Das Schlauchset umfasst weiterhin eine Bypass-Leitung, die den in die Pumpe eingelegten Schlauchabschnitt der Schlauchleitung überbrückt. In der Bypass-Leitung befindet sich ein Rückschlagventil, das den Druck im Schlauchset auf ein bestimmtes Maß begrenzt. Eine Infusionsleitung mit einer Bypass-Leitung zur Druckbegrenzung ist auch aus der US-A-4,391,599 bekannt.

Der Erfindung liegt die Aufgabe zu Grunde, ein Schlauchset für eine Vorrichtung zur Blutbehandlung, insbesondere eine extrakorporale Blutbehandlungsvorrichtung oder eine Vorrichtung zur Peritonealdialyse zu schaffen, das einen für den Patienten sicheren Betrieb der Blutbehandlungsvorrichtung erlaubt. Darüber hinaus ist eine Aufgabe der Erfindung eine Blutbehandlungsvorrichtung, insbesondere eine extrakorporale Blutbehandlungsvorrichtung oder eine Vorrichtung zur Peritonealdialyse zu schaffen, die eine erhöhte Sicherheit für den Patienten bietet.

Die Lösung dieser Aufgaben erfolgt erfindungsgemäß mit den Merkmalen der Patentansprüche 1 und 10. Vorteilhafte Ausführungsformen der Erfindung sind Gegenstand der Unteransprüche.

Das erfindungsgemäße Schlauchset für eine Blutbehandlungsvorrichtung, insbesondere eine extrakorporale Blutbehandlungsvorrichtung oder eine Vorrichtung zur Peritonealdialyse, zeichnet sich durch eine Einrichtung zum Auffangen von Partikeln aus, die in einem Schlauchleitungsabschnitt der Schlauchleitung des Schlauchsets vorgesehen ist, der stromab des in die okkludierende Schlauchpumpe einzulegenden Schlauchabschnitts liegt. Die Einrichtung zum Auffangen von Partikeln verhindert, dass in der Dialysierflüssigkeit, dem Substituat oder der Peritoneallösung möglicherweise ausgefällte Stoffe oder andere feste Bestandteile zurückgehalten werden. Insofern ist das erfindungsgemäße Schlauchset insbesondere zur Verwendung bei den Vorrichtungen zur akuten Hämodialyse von Vorteil, bei denen die für die Herstellung der Dialysierflüssigkeit oder des Substituats erforderlichen Komponenten in Mehrkammerbeuteln bereitgestellt werden, deren Umverpackung infolge unsachgemäßer Lagerung oder Handhabung beschädigt werden könnte.

Das erfindungsgemäße Schlauchset verfügt neben der Einrichtung zum Auffangen von Partikeln auch über eine Einrichtung zur Begrenzung des Drucks in dem Schlauchleitungsabschnitt, der zwischen dem in die okkludierende Schlauchpumpe einzulegenden Schlauchabschnitt und der Einrichtung zum Auffangen von Partikeln liegt. Die Einrichtung zur Begrenzung des Überdrucks verhindert, dass sich bei einer verringerten oder unterbrochenen Flüssigkeitsströmung durch die Einrichtung zum Auffangen von Partikeln in der Schlauchleitung ein zu großer Überdruck aufbaut.

Wenn das erfindungsgemäße Schlauchset bei den bekannten Vorrichtungen zur akuten Hämodialyse Verwendung findet, kann es in einigen Fällen zusätzlich einen Schlauchabschnitt enthalten, der dem Heizen der mit dem Schlauchset geförderten Flüssigkeit dient. Dieser kann als Heizungsbeutel ausgeführt sein. Dieses Heizen erfolgt üblicherweise, um die Temperatur der Lösung in die Nähe der Körpertemperatur zu bringen. Der Schlauchabschnitt zum Heizen kann sich stromauf oder stromab der okkludierenden Pumpe befinden. Vorzugsweise befindet sich der Schlauchabschnitt zum Heizen stromauf der Schlauchpumpe. Die Einrichtung zur Begrenzung des Überdrucks verhindert, dass bei laufender okkludierender Schlauchpumpe ein Teil des Schlauchsets stromab der Schlauchpumpe bersten kann. Wenn der Heizungsbeutel also dennoch stromab der Pumpe angeordnet sein sollte, kann somit auch dieser aufgrund eines zu hohen Überdrucks nicht bersten.

Die Einrichtung zur Begrenzung des Überdrucks stellt bei Überschreiten eines vorgegebenen maximalen Überdrucks in einem Schlauchabschnitt, der stromab des in die okkludierende Schlauchpumpe einzulegenden Schlauchabschnitts und stromauf der Einrichtung zum Auffangen von Partikeln liegt, eine Flüssigkeitsverbindung zwischen einem Schlauchabschnitt, der stromab des in die okkludierende Schlauchpumpe einzulegenden Schlauchabschnitts liegt, und einem Schlauchabschnitt her, der stromauf des in die okkludierende Schlauchpumpe einzulegenden Schlauchabschnitts liegt. Dadurch wird bei Überschreiten des vorgegebenen maximalen Überdrucks eine Bypass-Leitung geschaffen, durch die bei laufender okkludierender Schlauchpumpe die Flüssigkeit zirkulieren kann. Bei Unterschreiten des vorgegebenen maximalen Überdrucks ist die Flüssigkeitsverbindung unterbrochen, so dass mit der okkludierenden Schlauchpumpe die Flüssigkeit gefördert werden kann.

Bei einer bevorzugten Ausführungsform weist die Einrichtung zur Begrenzung des Überdrucks ein vorgespanntes Rückschlagventil auf, das bei Überschreiten des vorgegebenen maximalen Überdrucks in Richtung der Flüssigkeitsströmung von dem Schlauchabschnitt stromab des in die okkludierende Schlauchpumpe einzulegenden Schlauchabschnitts zu dem Schlauchabschnitt stromauf des in die okkludierende Schlauchpumpe einzulegenden Schlauchabschnitts geöffnet ist. Bei Unterschreiten des vorgegebenen maximalen Überdrucks ist das Rückschlagventil geschlossen. Das Rückschlagventil kann an Schlauchstücken angeschlossen sein, die von der Schlauchleitung unmittelbar stromauf und stromab des in die okkludierende Schlauchpumpe einzulegenden Schlauchabschnitts abzweigen.

Bei einer alternativen Ausführungsform weist die Einrichtung zur Begrenzung des Überdrucks ein elektromagnetisch, hydraulisch oder pneumatisch betätigbares Ventil auf, das von einer Steuereinheit ansteuerbar ist, die mit einer Einrichtung zur Erkennung eines Störfalls auf Grund eines erhöhten Durchflusswiderstandes durch die Einrichtung zum Auffangen von Partikeln zusammenwirkt. Die Steuereinheit ist derart ausgebildet, dass das Ventil geöffnet ist, wenn ein Störfall erkannt wird, während das Ventil geschlossen ist, wenn kein Störfall erkannt wird. Auch das elektromagnetisch, hydraulisch oder pneumatisch betätigbare Ventil kann an Schlauchstücken angeschlossen sein, die von der Schlauchleitung abzweigen.

Die Einrichtung zum Auffangen von Partikeln ist vorzugsweise ein Partikelfilter, der eine Porengröße von 0,1 µm bis 100 µm, vorzugsweise 0,2 µm bis 50 µm, besonders bevorzugt 0,2 µm bis 20 µm aufweist. Derartige Partikelfilter als solche gehören zum Stand der Technik. Der Partikelfilter kann fest mit den Schlauchabschnitten der Schlauchleitung verbunden oder mit entsprechenden Anschlussstücken an die Schlauchleitungsabschnitte angeschlossen sein.

Das erfindungsgemäße Schlauchset findet zusammen mit einem Beutel zur Bereitstellung von Dialysierflüssigkeit oder Substituat für eine extrakorporale Blutbehandlung oder zur Bereitstellung einer Peritoneallösung für eine Peritonealdialyse Verwendung. Der Beutel kann über entsprechende Anschlussstücke mit der Schlauchleitung verbunden werden. Es ist aber auch möglich, dass Beutel und Schlauchleitung bereits fest miteinander verbunden sind.

Der Beutel zur Bereitstellung von Dialysierflüssigkeit, Substituat oder Peritoneallösung ist vorzugsweise ein Mehrkammerbeutel, dessen Kammern mit verschiedenen Komponenten zur Herstellung der jeweiligen Flüssigkeit befüllt sind. Insbesondere ist der Beutel ein Zweikammerbeutel, dessen Kammern von einer auftrennbaren Naht voneinander getrennt sind.

Wenn das erfindungsgemäße Schlauchset für eine extrakorporalen Blutbehandlung Verwendung findet, kann das Schlauchset zum Fördern der Dialysierflüssigkeit eingesetzt werden, wobei die Schlauchleitung in die Dialysierflüssigkeitspumpe eingelegt wird. Das erfindungsgemäße Schlauchset kann bei der extrakorporalen Blutbehandlung aber auch zum Fördern des Substituats verwendet werden, wobei die Schlauchleitung in die Substituatpumpe eingelegt wird. Bei der Verwendung des Schlauchsets für die Peritonealdialyse wird die Schlauchleitung des Schlauchsets in die okkludierende Schlauchpumpe zur Förderung der Peritoneallösung eingelegt.

Eine bevorzugte Ausführungsform der Vorrichtung zur Blutbehandlung, bei der es sich um eine Vorrichtung zur extrakorporalen Blutbehandlung oder eine Vorrichtung zur Peritonealdialyse handeln kann, sieht eine Einrichtung zur Erkennung eines Störfalls auf Grund einer verringerten oder unterbrochenen Flüssigkeitsströmung durch die Einrichtung zum Auffangen von Partikeln vor. Die Einrichtung zur Erkennung eines Störfalls wirkt bei einer besonders bevorzugten Ausführungsform mit einer Alarmeinheit zusammen, die bei einem Störfall einen optischen und/oder akustischen und/oder taktilen Alarm gibt, um den Anwender darauf aufmerksam zu machen, dass zur Vermeidung eines zu hohen Überdrucks in der Schlauchleitung die Flüssigkeit bei laufender okkludierender Pumpe nur in der Bypass-Leitung zirkuliert.

Die Einrichtung zur Erkennung eines Störfalls weist bei einer ersten bevorzugten Ausführungsform eine Auswerteinheit und einen Druckaufnehmer auf, der den Druck in dem Schlauchabschnitt misst, der zwischen dem in die okkludierende Schlauchpumpe einzulegenden Schlauchabschnitt und der Einrichtung zum Auffangen von Partikeln liegt. Die Auswerteinheit ist derart ausgebildet, dass der mit dem Druckaufnehmer gemessene Druck mit einem vorgegebenen maximalen Überdruck verglichen wird. Wenn der vorgegebene maximale Überdruck überschritten wird, erkennt die Auswerteinheit den Störfall. Dann wird das elektromagnetisch oder pneumatisch betätigbare Ventil in der Bypass-Leitung von der Steuereinheit geöffnet, so dass die Flüssigkeit bei laufender okkludierender Pumpe zirkulieren kann.

Eine alternative Ausführungsform der Einrichtung zur Erkennung eines Störfalls sieht eine Auswerteinheit und eine Einrichtung zum Wiegen eines Beutels zur Aufnahme von Dialysierflüssigkeit, Substituat oder Peritoneallösung vor. Eine derartige Einrichtung zum Wiegen ist bei den bekannten Blutbehandlungsvorrichtungen zur Bilanzierung der Flüssigkeiten ohnehin vorhanden. Die Auswerteinheit ist derart ausgebildet, dass die Gewichtsabnahme des Beutels innerhalb eines vorgegebenen Zeitintervalls mit einem vorgegebenen Grenzwert verglichen wird. Der vorgegebene Grenzwert entspricht der zu erwartenden Gewichtsabnahme des Beutels, wenn die Flüssigkeit mit der gewünschten Förderrate gefördert wird. Wenn die Flüssigkeitsströmung durch die Einrichtung zum Auffangen von Partikeln verringert oder unterbrochen ist, d.h. sich der Partikelfilter teilweise oder vollständig zugesetzt hat, unterschreitet die Gewichtsabnahme den vorgegebenen Grenzwert. Dann erkennt die Auswerteinheit den Störfall.

Im Folgenden werden Ausführungsbeispiele der Erfindung unter Bezugnahme auf die Zeichnungen näher erläutert.

Es zeigen:
- Fig. 1: ein Ausführungsbeispiel des erfindungsgemäßen Schlauchsets für eine Blutbehandlungsvorrichtung in schematischer Darstellung,
- Fig. 2: ein Ausführungsbeispiel einer erfindungsgemäßen Vorrichtung zur extrakorporalen Blutbehandlung mit einem erfindungsgemäßen Schlauchset in stark vereinfachter schematischer Darstellung,
- Fig. 3: ein zweites Ausführungsbeispiel einer erfindungsgemäßen Vorrichtung zur extrakorporalen Blutbehandlung mit einem erfindungsgemäßen Schlauchset in stark vereinfachter schematischer Darstellung,
- Fig. 4: ein weiteres Ausführungsbeispiel der extrakorporalen Blutbehandlungsvorrichtung mit einem erfindungsgemäßen Schlauchset in stark vereinfachter schematischer Darstellung und
- Fig. 5: ein Ausführungsbeispiel einer erfindungsgemäßen Vorrichtung zur Peritonealdialyse mit einem erfindungsgemäßen Schlauchset in stark vereinfachter schematischer Darstellung.

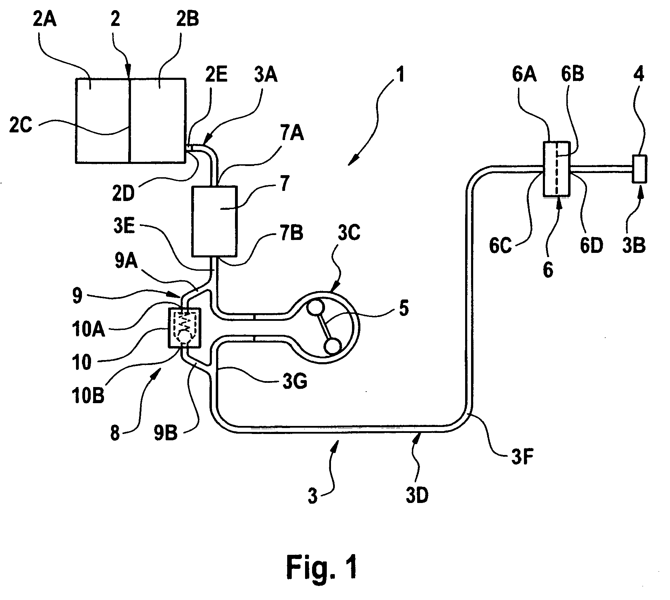

Fig. 1 zeigt in schematischer Darstellung ein Ausführungsbeispiel des erfindungsgemäßen Schlausets, das bei dem vorliegenden Ausführungsbeispiel zur Verwendung in einer Vorrichtung zur akuten Hämodialyse bestimmt ist. Das Schlauchset 1 umfasst einen Beutel 2 zur Bereitstellung der für die Herstellung der Dialysierflüssigkeit oder des Substituats erforderlichen Komponenten und eine Schlauchleitung 3. Bei dem vorliegenden Ausführungsbeispiel sind Beutel 2 und Schlauchleitung 3 fest miteinander verbunden. Es ist aber auch möglich, dass der Beutel 2 nicht Teil des Schlauchsets ist, sondern Beutel 2 und Schlauchleitung 3 mit entsprechenden Konnektoren miteinander verbunden werden können.

Bei dem Beutel 2 handelt es sich um einen Zweikammerbeutel, der eine erste Kammer 2A und eine zweite Kammer 2B aufweist, die von einer auftrennbaren Naht 2C voneinander getrennt sind. Die erste Kammer 2A des Beutels 2 enthält eine bikarbonathaltige Lösung, während die zweite Kammer 2B eine typischerweise saure, calciumhaltige Lösung enthält. Beide Lösungen werden erst kurz vor der Behandlung nach Auftrennen der Naht 2C miteinander vermischt.

Das eine Ende 3A der Schlauchleitung 3 ist an einem Auslass 2D des Beutels 2 angeschlossen. Der Auslass 2D des Beutels 2 ist mit einem Verschlussteil 2E verschlossen, der erst kurz vor der Behandlung geöffnet wird. Das andere Ende 3B der Schlauchleitung 3 weist ein Anschlussstück 4 auf, um die Schlauchleitung an ein entsprechendes Anschlussstück an dem Einlass der Dialysierflüssigkeitskammer eines Dialysators oder an eine Tropfkammer anschließen zu können.

Die Schlauchleitung 3 gliedert sich in mehrere Schlauchabschnitte. Die Schlauchleitung 3 weist einen Schlauchabschnitt 3C auf, der in eine okkludierende Schlauchpumpe 5, bei dem vorliegenden Ausführungsbeispiel in die Dialysierflüssigkeitspumpe oder Substituatpumpe einer Vorrichtung zur akuten Härnodialyse eingelegt werden kann. Die Dialysierflüssigkeits- oder Substituatpumpe 5 der extrakorporalen Blutbehandlungsvorrichtung ist in Fig. 1 nur andeutungsweise dargestellt.

In dem Schlauchabschnitt 3D der Schlauchleitung, der stromab des in die okkludierende Schlauchpumpe 5 einzulegenden Schlauchabschnitts 3C liegt, befindet sich eine Einrichtung 6 zum Auffangen von Partikeln. Bei dieser Einrichtung handelt es sich um einen im Stand der Technik als solchen bekannten Partikelfilter 6. Der Partikelfilter 6 weist ein Gehäuse 6A auf, das beispielsweise aus Polycarbonat besteht. In dem Gehäuse befindet sich eine Filtermembran 6B mit einer Porengröße von 0,1 bis 100 µm, vorzugsweise 0,2 bis 50 µm, besonders bevorzugt 0,2 bis 20 µm. Die Membran kann aus Polyamid bestehen. Das Filtergehäuse 6A weist einen Einlass 6C und einen Auslass 6D auf, die mit den zu dem Filter 6 hinführenden bzw. von dem Filter wegführenden Schlauchleitungsabschnitten fest verbunden sind.

Das Schlauchset 1 verfügt bei dem Ausführungsbeispiel von Fig. 1 über einen Beutel 7, der zum Aufheizen der Dialysierflüssigkeit auf die Körpertemperatur des Patienten dient. Der Heizbeutel 7 befmdet sich vorzugsweise in dem Schlauchabschnitt 3E, der zwischen dem Beutel 2 und dem in die Schlauchpumpe 5 einzulegenden Schlauchabschnitt 3C liegt. Folglich ist der Heizbeutel 7 stromauf der Schlauchpumpe 5 angeordnet. Der Heizbeutel kann aber auch in dem Schlauchabschnitt 3F stromab des in die Schlauchpumpe 5 einzulegenden Schlauchabschnitts 3C und stromauf des Partikelfilters 6 angeordnet sein. Der Heizbeutel weist einen Einlass 7A und einen Auslass 7B auf, der mit den entsprechenden Schlauchabschnitten fest verbunden ist. Zum Aufheizen der Dialysierflüssigkeit oder des Substituats auf die Körpertemperatur wird der Heizbeutel 7 auf eine nicht dargestellte Heizeinrichtung der Vorrichtung zur akuten Hämodialyse aufgelegt.

Der Partikelfilter 6 hält Partikel zurück, die bei einer unsachgemäßen Lagerung oder Beschädigung des nicht vollständig dargestellten Verpackungssystems ausfällen können. Wenn sich der Partikelfilter 6 teilweise oder vollständig zugesetzt haben sollte, so dass der Durchflusswiderstand des Partikelfilters relevant gestiegen ist, kann sich bei laufender Schlauchpumpe 5 ein Überdruck in dem Schlauchabschnitt 3F aufbauen, der zwischen der Schlauchpumpe 5 und dem Partikelfilter 6 liegt. Daher verfügt das Schlauchset 1 über eine Einrichtung 8 zur Begrenzung des Überdrucks in dem Schlauchleitungsabschnitt 3F zwischen Schlauchpumpe 5 und Partikelfilter 6.

Die Einrichtung 8 zur Begrenzung des Überdrucks weist eine Bypass-Leitung 9 und ein Rückschlagventil 10 auf, das in der Bypass-Leitung 9 angeordnet ist. Die Bypass-Leitung 9 umfasst zwei Schlauchstücke 9A und 9B. Das eine Schlauchstück 9A zweigt von dem Schlauchabschnitt 3E ab, der stromauf des in die Schlauchpumpe 5 einzulegenden Schlauchabschnitts 3C liegt, und ist an dem einen Anschluss 10A des Rückschlagventils 10 angeschlossen. Das andere Schlauchstück 9B ist an dem anderen Anschluss 10 B des Rückschlagventils 10 angeschlossen und mündet in den Schlauchabschnitt 3 G, der zwischen dem in die Schlauchpumpe 5 einzulegenden Schlauchabschnitt 3C und dem Heizbeutel 7 liegt.

Das Rückschlagventil 10 ist vorzugsweise ein federbelastetes Kugelrückschlagventil, das sich bei Überschreiben eines maximalen vorgegebenen Überdrucks in dem Schlauchabschnitt stromab der Pumpe 5 und stromauf des Filters 6 in Richtung des Beutels 2 öffnet, ansonsten aber geschlossen ist. Für den Fall, dass sich das Partikelfilter 6 zusetzen sollte, öffnet das Rückschlagventil 10 bei laufender Schlauchpumpe 5, so dass die Flüssigkeit über die Bypass-Leitung 9 zirkuliert. Dadurch wird verhindert, dass sich der Überdruck bei laufender Pumpe weiter erhöht, wodurch die Gefahr des Berstens von Komponenten stromab der laufenden Pumpe, insbesondere von Komponenten der Einrichtung zum Auffangen von Partikeln, besteht.

Das erfindungsgemäße Schlauchset 1 kann neben dem Beutel 2 zur Aufnahme der Dialysierflüssigkeit oder des Substituats und dem Heizbeutel 7 noch weitere Komponenten aufweisen, die der besseren Übersichtlichkeit halber nicht dargestellt sind. Beispielsweise kann das Schlauchset weitere Anschlussstücke enthalten. Auch kann eine Tropfkammer Bestandteil des Schlauchsets sein, wenn das Schlauchset zum Zuführen von Substituat verwendet wird. Das Schlauchset kann aber auch an eine Tropfkammer angeschlossen werden, die in dem Schlauset des extrakorporalen Blutkreislaufs vorgesehen ist.

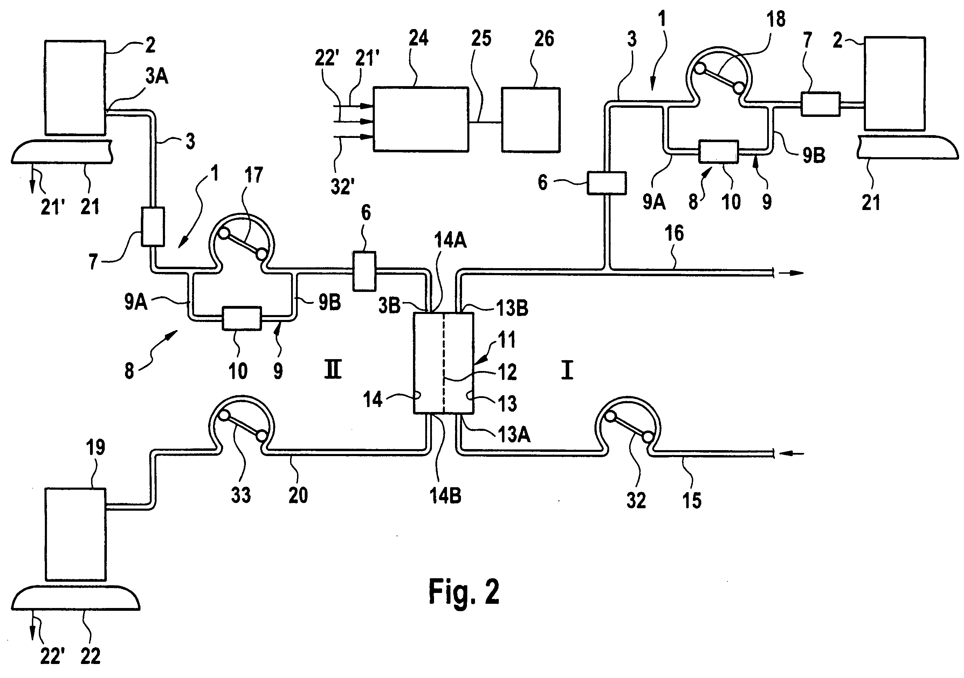

Fig. 2 zeigt die wesentlichen Komponenten einer extrakorporalen Blutbehandlungsvorrichtung mit dem Schlauchset. Bei dem vorliegenden Ausführungsbeispiel handelt es sich um eine Vorrichtung zur akuten Hämodialyse, die sich dadurch auszeichnet, dass Dialysierflüssigkeit und/oder Substituat in Beuteln bereitgestellt werden. Da eine Vorrichtung zur akuten Hämodialyse zum Stand der Technik gehört, zeigt Fig. 2 nur in stark vereinfachter schematischer Darstellung die wesentlichen Komponenten der Vorrichtung.

Die extrakorporale Blutbehandlungsvorrichtung weist als Austauscheinheit einen Dialysator 11 auf, der durch eine semipermeable Membran 12 in eine Blutkammer 13 und eine Dialysierflüssigkeitskammer 14 getrennt ist. Die Blutkammer 13 ist Teil eines extrakorporalen Blutkreislaufs I, während die Dialysierflüssigkeitskammer 14 Teil eines Flüssigkeitssystems II der extrakorporalen Blutbehandlungsvorrichtung ist.

Eine arterielle Blutleitung 15 führt von dem Patienten zu einem Einlass 13A der Blutkammer 13. Zum Fördern des Bluts dient eine Blutpumpe 32. Von dem Auslass 13B der Blutkammer 13 führt eine venöse Blutleitung 16 zu dem Patienten.

Bei dem vorliegenden Ausführungsbeispiel der Vorrichtung zur akuten Hämodialyse im weiteren Sinn findet das unter Bezugnahme auf Fig. 1 beschriebene Schlauchset sowohl zum Fördern von Dialysierflüssigkeit als auch Substituat Verwendung, weshalb das Behandlungsverfahren treffender auch als Hämodiafiltration bezeichnet werden kann. Daher werden für das Schlauchset 1 die gleichen Bezugzeichen verwendet. Zur Bereitstellung von Dialysierflüssigkeit ist der Beutel 2 des Schlauchsets 1 mit den für die Herstellung der Dialysierflüssigkeit erforderlichen Komponenten befüllt, während zur Bereitstellung von Substituat der Beutel 2 mit den zur Herstellung des Substituats erforderlichen Komponenten befüllt ist. Es können die im Wesentlichen gleichen Komponenten wie zur Herstellung der Dialysierflüssigkeit sein. Insofern unterscheiden sich in diesem Ausführungsbeispiel beide Schlauchsets nicht voneinander.

Das freie Ende 3B der Schlauchleitung 3 des Schlauchsets 1 für die Dialysierflüssigkeit ist an dem Einlass 14A der Dialysierflüssigkeitskammer 14 des Dialysators 11 angeschlossen, während das freie Ende 3B der Schlauchleitung 3 des Schlauchsets 1 für das Substituat an der venösen Blutleitung 16 angeschlossen ist. Das freie Ende 3B der Schlauchleitung 3 kann auch an der arteriellen Schlauchleitung 15 angeschlossen sein. Der besseren Übersichtlichkeit halber wird auf die Darstellung entsprechender Anschlussstücke verzichtet. Auch ist eine Tropfkammer nicht dargestellt. Dabei wird die Schlauchleitung 3 in eine Dialysierflüssigkeitspumpe 17 zum Fördern der Dialysierflüssigkeit bzw. eine Substituatpumpe 18 zum Fördern von Substituat eingelegt, die Bestandteil der Blutbehandlungsvorrichtung sind.

Die beiden Partikelfilter 6 halten möglicherweise ausfällende Partikel wirkungsvoll zurück. Für den Fall, dass sich einer der beiden Partikelfilter 6 zusetzen sollte, öffnet sich das jeweilige Rückschlagventil 10, so dass die Dialysierflüssigkeit bzw. das Dialysat durch die Bypass-Leitung 9 zirkulieren kann, ohne dass der Druck in der jeweiligen Schlauchleitung über einen vorgegebenen maximalen Grenzwert ansteigt.

Die verbrauchte Dialysierflüssigkeit wird in einem Beutel 19 aufgefangen, der über eine Schlauchleitung 20 an dem Auslass 14B der Dialysierflüssigkeitskammer 14 angeschlossen ist. Die Schlauchleitung 20 wird üblicherweise in eine Schlauchrollenpumpe 33 (Effluentpumpe) eingelegt, die stromab der Dialysierflüssigkeitskammer 14 angeordnet ist.

Des Weiteren verfügt die extrakorporale Blutbehandlungsvorrichtung über eine Einrichtung zum Wiegen der Beutel 2, 19, die eine gemeinsame Waage 21 für die Beutel 2 für frische Dialysierflüssigkeit bzw. Substituat und eine Waage 22 für den Beutel 19 für verbrauchte Dialysierflüssigkeit umfasst, auf denen die Beutel liegen. Anstelle einer gemeinsamen Waage für frische Dialysierflüssigkeit bzw. Substituat können aber auch getrennte Waagen vorgesehen sein. Die Blutpumpe 32, die Schlauchrollenpume 33 (Effluentpumpe), die Dialysierflüssigkeitspumpe 17 und die Substituatpumpe 18 werden von einer zentralen Steuereinheit 24 über nicht dargestellte Steuerleitungen angesteuert, die über Datenleitungen 21', 22'die Messwerte der Waagen 21, 22 empfängt.

Die zentrale Steuer- und Recheneinheit 24 ist über eine Datenleitung 25 mit einer Alarmeinheit 26 verbunden, die in einem Störfall einen optischen und/oder akustischen und/oder taktilen Alarm gibt.

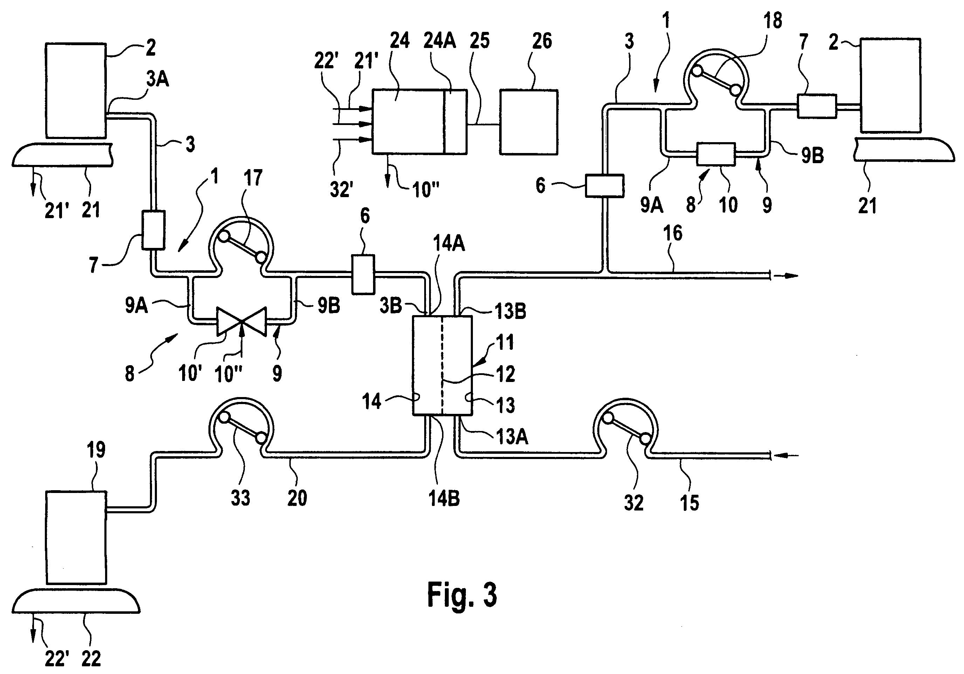

Fig. 3 zeigt ein zweites Ausführungsbeispiel der extrakorporalen Blutbehandlungsvorrichtung in stark vereinfachter schematischer Darstellung. Die einander entsprechenden Teile sind mit den gleichen Bezugszeichen versehen.

Die extrakorporale Blutbehandlungsvorrichtung von Fig. 3 weist ein Schlauchset 1 für Dialysierflüssigkeit auf, bei dem anstelle eines Rückschlagventils 10 in der Bypass-Leitung 9 ein elektromagnetisch betätigbares Absperrorgan 10' vorgesehen ist. Darüber hinaus weist die Blutbehandlungsvorrichtung von Fig. 2 eine Einrichtung zur Erkennung eines Störfalls auf Grund einer verringerten oder unterbrochenen Flüssigkeitsströmung durch den Partikelfilter 6 und eine Steuereinheit zum Ansteuern des elektromagnetisch betätigbaren Absperrorgans 10' auf. Die Einrichtung zur Erkennung des Störfalls verfügt über eine Auswerteinheit und eine Einrichtung zum Wiegen des Beutels für die Dialysierflüssigkeit, bei der es sich um die Waage 21 zum Wiegen des Beutels 2 handelt. Die Auswerteinheit 24A ist Teil der zentralen Steuer- und Recheneinheit 24 der Blutbehandlungsvorrichtung, mit der das elektromagnetische Absperrorgan 10' über eine Steuerleitung 10" verbunden ist. Die Auswerteinheit 24A der zentralen Steuer- und Recheneinheit 24 überwacht während der Blutbehandlung die Gewichtsabnahme des Beutels 2, in der sich die Dialysierflüssigkeit befindet. Die Gewichtsabnahme der Dialysierflüssigkeit in einem vorgegebenen Zeitintervall vergleicht die Auswerteinheit mit einem vorgegebenen Grenzwert. Bei Unterschreiten des vorgegebenen Grenzwertes schließt die Auswerteinheit darauf, dass die Flüssigkeitsströmung für den Partikelfilter 6 verringert oder unterbrochen ist, so dass sich bei laufender Dialysierflüssigkeitspumpe 17 ein unzulässig hoher Überdruck in der Schlauchleitung 2 aufbaut. Wenn dieser Störfall erkannt ist, erzeugt die Steuer- und Recheneinheit 24 ein Alarmsignal, das die Alarmeinheit 26 empfängt, die über die Datenleitung 25 mit der Steuer- und Recheneinheit 24 verbunden ist. Die Alarmeinheit gibt dann einen optischen und/oder akustischen und/oder taktilen Alarm. Gleichzeitig öffnet die Steuer- und Recheneinheit 24 das elektromagnetische Absperrorgan 10' in der Bypass-Leitung 9, so dass die Dialysierflüssigkeit zirkulieren kann. Der vorgegebene Grenzwert für die Gewichtsabnahme pro Zeiteinheit sollte so gewählt sein, dass die Bypass-Leitung geöffnet wird, wenn der vorgegebene maximale Überdruck in der Schlauchleitung überschritten wird.

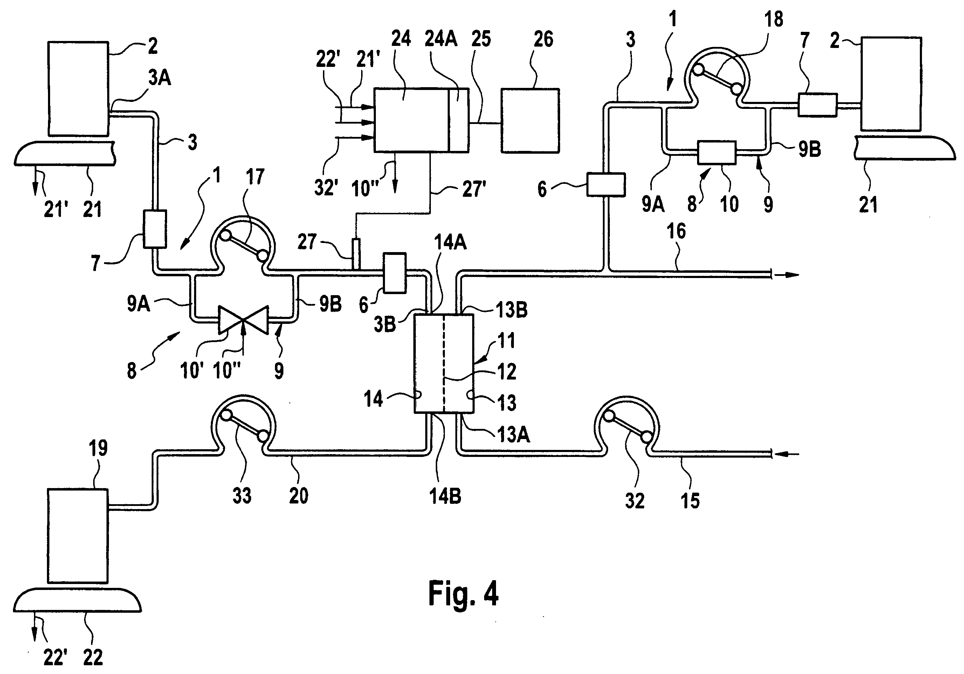

Fig. 4 zeigt ein weiteres Ausführungsbeispiel der extrakorporalen Blutbehandlungsvorrichtung, die sich von der unter Bezugnahme auf die in Fig. 3 beschriebenen Blutbehandlungsvorrichtung dadurch unterscheidet, dass nicht die Gewichtsabnahme des Beutels 2, sondern der Druck in der Schlauchleitung 3 des Schlauchsets 1 gemessen wird. Die einander entsprechenden Teile sind wieder mit den gleichen Bezugszeichen versehen.

Bei der Blutbehandlungsvorrichtung von Fig. 4 ist in dem Schlauchset 1 für die Dialysierflüssigkeit ein Druckaufnehmer 27 vorgesehen, der in dem Schlauchabschnitt 3F zwischen dem in die Blutpumpe 5 einzulegenden Schlauchabschnitt 3C und dem Partikelfilter 6 angeordnet ist. Der Druckaufnehmer 27 ist über eine Datenleitung 27' mit der zentralen Steuer- und Recheneinheit 24 verbunden. Für den Fall, dass mit dem Druckaufnehmer 27 ein Druck gemessen wird, der den vorgegebenen maximalen Überdruck überschreitet, wird der Störfall erkannt und Alarm gegeben. Die Gewichtsabnahme des Beutels 2 braucht bei diesem Ausführungsbeispiel nicht überwacht zu werden, um einen erhöhten Strömungswiderstand durch den Partikelfilter zu erkennen.

Die Figuren 2 bis 4 zeigen Ausführungsbeispiele, bei denen das Schlauchset 1 für das Substituat nicht über ein elektromagnetisch oder pneumatisch betätigbares Absperrorgan 10', sondern ein Rückschlagventil 10 verfügt. Es ist aber auch möglich, dass das Schlauchset für das Substituat über ein elektromagnetisch, hydraulisch oder pneumatisch betätigbares Absperrorgan verfügt, das von der zentralen Steuer- und Recheneinheit 24 angesteuert wird.

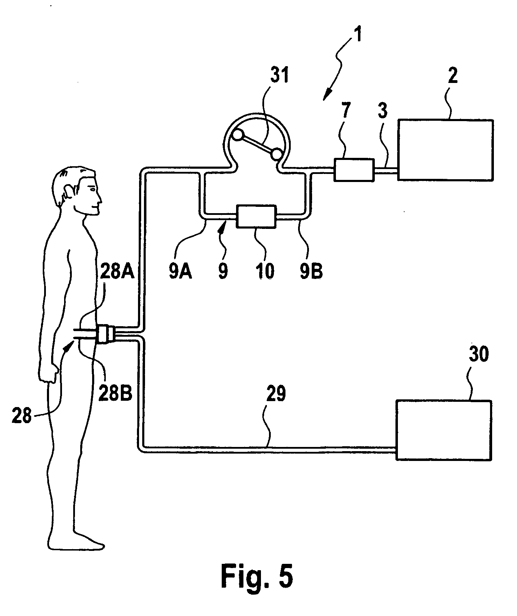

Fig. 5 zeigt in stark vereinfachter schematischer Darstellung eine Vorrichtung zur Peritonealdialyse, bei der das unter Bezugnahme auf Fig. 1 beschriebene Schlauchset 1 auch Verwendung finden kann. Für die Verwendung des Schlauchsets bei der Peritonealdialyse ist der Beutel 2 aber nicht mit den für die Herstellung einer Dialysierflüssigkeit, sondern einer Peritoneallösung erforderlichen Komponenten befüllt. Ansonsten unterscheiden sich die Schlauchsets aber nicht voneinander. Die einander entsprechenden Teile sind daher wieder mit den gleichen Bezugszeichen versehen. Die Vorrichtung zur Peritonealdialyse umfasst das erfindungsgemäße Schlauchset 1, wobei das freie Ende 3B der Schlauchleitung 3 an das erste Lumen 28A eines zweilumigen Peritonealkatheters 28 zum Zuführen von Peritoneallösung in den Peritonealraum bzw. Abführen der Lösung aus dem Peritonealraum des Patienten angeschlossen ist. An das zweite Lumen 28B des Peritonealkatheters 28 ist eine Schlauchleitung 29 angeschlossen, die zu einem Beutel 30 zum Auffangen von Peritoneallösung führt. Die Schlauchleitung 2 des Schlauchsets wird in eine okkludierende Schlauchpumpe 31 der Vorrichtung zur Peritonealdialyse eingelegt.

Der Partikelfilter 6 des Schlauchsets 1 hält wieder ausfällende Partikel zurück, die bei unsachgemäßer Lagerung oder Beschädigung des Verpackungssystems des Beutels 2 in der Peritoneallösung enthalten sein können. Auch wird hier wieder das Übersteigen eines maximal vorgegebenen Überdrucks bei laufender Schlauchpumpe verhindert, wenn sich der Partikelfilter zusetzen sollte.

## Patentansprüche

1. Schlauchset für eine Blutbehandlungsvorrichtung mit einer Schlauchleitung 3, die einen in eine okkludierende Schlauchpumpe der Blutbehandlungsvorrichtung einzulegenden Schlauchabschnitt (3C) aufweist, **dadurch gekennzeichnet, dass** in einem Schlauchabschnitt (3D) stromab des in die okkludierende Schlauchpumpe einzulegenden Schlauchabschnitts eine Einrichtung zum Auffangen von Partikeln (6) vorgesehen ist, wobei eine Einrichtung (8) zur Begrenzung des Überdrucks vorgesehen ist, die derart ausgebildet ist, dass bei Überschreiten eines vorgegebenen maximalen Überdrucks in einem Schlauchabschnitt, der stromab des in die okkludierende Schlauchpumpe einzulegenden Schlauchabschnitts (3C) und stromauf der Einrichtung (6) um Auffangen von Partikeln liegt, eine Flüssigkeitsverbindung zwischen einem Schlauchabschnitt (3G), der stromab des in die okkludierende Schlauchpumpe einzulegenden Schlauchabschnitts liegt, und einem Schlauchabschnitt (3E), der stromauf des in die okkludierende Schlauchpumpe einzulegenden Schlauchabschnitts liegt, hergestellt wird, und bei Unterschreiten des vorgegebenen maximalen Überdrucks die Flüssigkeitsverbindung unterbrochen ist.

2. Schlauchset nach Anspruch 1, **dadurch gekennzeichnet, dass** die Einrichtung (8) zur Begrenzung des Überdrucks ein vorgespanntes Rückschlagventil (10) aufweist, das bei Überschreiten des vorgegebenen maximalen Überdrucks in Richtung der Flüssigkeitsströmung von dem stromab des in die okkludierende Schlauchpumpe einzulegenden Schlauchabschnitts (3C) zu dem stromauf des in die okkludierende Schlauchpumpe einzulegenden Schlauchabschnitts geöffnet ist und bei Unterschreiten des vorgegebenen maximalen Überdrucks geschlossen ist.

3. Schlauchset nach Anspruch 2, **dadurch gekennzeichnet, dass** von dem Schlauchabschnitt (3G), der stromab des in die okkludierende Schlauchpumpe einzulegenden Schlauchabschnitts (3C) liegt, ein erstes Schlauchstück (9B) abzweigt, dessen Ende mit dem einen Anschluss (10B) des vorgespannten Rückschlagventils (10) verbunden ist, und dass von dem Schlauchabschnitt (3E), der stromauf des in die okkludierende Schlauchpumpe einzulegenden Schlauchabschnitts (3C) liegt, ein zweites Schlauchstück (9A) abzweigt, dessen Ende mit dem anderen Anschluss (10A) des vorgespannten Rückschlagventils (10) verbunden ist.

4. Schlauchset nach Anspruch 1, **dadurch gekennzeichnet, dass** die Einrichtung (8) zur Begrenzung des Überdrucks ein elektromagnetisch oder pneumatisch betätigbares Ventil (10') aufweist, das von einer Steuereinheit ansteuerbar ist, die mit einer Einrichtung zur Erkennung eines Störfalls aufgrund eines erhöhten Durchflusswiderstandes durch die Einrichtung zum Auffangen von Partikeln zusammenwirkt, wobei die Steuereinheit derart ausgebildet ist, dass das Ventil geöffnet ist, wenn ein Störfall erkannt wird, so dass eine Verbindung zwischen dem Schlauchabschnitt, der stromab des in die okkludierende Schlauchpumpe einzulegenden Schlauchabschnitts liegt, und dem Schlauchabschnitt, der stromauf des in die okkludierende Schlauchpumpe einzulegenden Schlauchabschnitts liegt, hergestellt wird, und das Ventil geschlossen ist, wenn kein Störfall erkannt wird.

5. Schlauchset nach Anspruch 4, **dadurch gekennzeichnet, dass** von dem Schlauchabschnitt (3G), der stromab des in die okkludierende Schlauchpumpe einzulegenden Schlauchabschnitts (3C) liegt, ein erstes Schlauchstück (9B) abzweigt, dessen Ende mit dem einen Anschluss (10B) des elektromagnetisch, hydraulisch oder pneumatisch betätigbaren Ventils (10') verbunden ist, und dass von dem Schlauchabschnitt (3E), der stromauf des in die okkludierende Schlauchpumpe einzulegenden Schlauchabschnitts (3C) liegt, ein zweites Schlauchstück (9A) abzweigt, dessen Ende mit dem anderen Anschluss (10A) des elektromagnetisch oder pneumatisch betätigbaren Ventils (10') verbunden ist.

6. Schlauchset nach einem der Ansprüche 1 bis 5, **dadurch gekennzeichnet, dass** die Einrichtung zum Auffangen von Partikeln ein Partikelfilter (6) ist, der eine Porengröße von 0,1 µm bis 100 µm, vorzugsweise 0,2 µm bis 50 µm, besonders bevorzugt 0,2 µm bis 20 µm aufweist.

7. Schlauchset nach einem der Ansprüche 1 bis 6, **dadurch gekennzeichnet, dass** das Schlauchset (1) einen Beutel (7) zum Beheizen der durch die Schlauchleitung strömenden Flüssigkeit aufweist.

8. Anordnung mit einem Beutel (2) zur Bereitstellung von Dialysierflüssigkeit oder Substituat für eine extrakorporale Blutbehandlung oder einer Peritoneallösung für eine Peritonealdialyse und einem Schlauchset (1) nach einem der Ansprüche 1 bis 7.

9. Anordnung nach Anspruch 8, **dadurch gekennzeichnet, dass** der Beutel (2) zur Bereitstellung von Dialysierflüssigkeit, Substituat oder Peritoneallösung ein Mehrkammerbeutel ist, dessen Kammern (2A,2B) mit verschiedenen Komponenten zur Herstellung der Dialysierflüssigkeit, des Substituats oder der Peritoneallösung befüllt sind.

10. Vorrichtung zur Blutbehandlung mit einem Schlauchset nach einem der Ansprüche 1 bis 7.

11. Vorrichtung zur Blutbehandlung nach Anspruch 10, **dadurch gekennzeichnet, dass** die Blutbehandlungsvorrichtung eine Vorrichtung zur Peritoneldialyse mit einer Leitung (3) zum Zuführen einer Peritoneallösung und einer Leitung (29) zum Abführen der Peritoneallösung ist, und dass die Schlauchleitung (3) des Schlauchsets (1) nach einem der Ansprüche 1 bis 7 die Leitung zum Zuführen der Peritonellösung ist, wobei die Schlauchleitung (3) des Schlauchsets (1) in eine okkludierende Schlauchpumpe (31) der Vorrichtung zur Peritonealdialyse zur Förderung der Peritoneallösung eingelegt ist.

12. Vorrichtung zur Blutbehandlung nach Anspruch 11, **dadurch gekennzeichnet, dass** an ein Ende (3A) der Schlauchleitung (3) des Schlauchsets (1) ein Beutel (2) zur Bereitstellung von Peritoneallösung angeschlossen ist, der ein Mehrkammerbeutel ist, dessen Kammern (2A, 2B) verschiedene Komponenten zur Herstellung der Peritoneallösung enthalten.

13. Vorrichtung zur Blutbehandlung nach Anspruch 10, **dadurch gekennzeichnet, dass** die Blutbehandlungsvorrichtung eine Vorrichtung zur extrakorporalen Blutbehandlung mit einer Austauscheinheit (11) ist, die eine erste Kammer (13) aufweist, die Teil eines extrakorporalen Blutkreislaufs (I) ist, und eine zweite Kammer (14) aufweist, die Teil eines Flüssigkeitssystems (II) ist, wobei der extrakorporale Blutkreislauf (I) eine zu einem Einlass (13A) der ersten Kammer (13) der Austauscheinheit (11) führende arterielle Blutleitung (15) und eine von einem Auslass (13B) der ersten Kammer (13) der Austauscheinheit (11) abgehende venöse Blutleitung (16) umfasst.

14. Vorrichtung nach Anspruch 13, **dadurch gekennzeichnet, dass** an das eine Ende (3A) der Schlauchleitung (3) des Schlauchsets (1) ein Beutel (2) zur Bereitstellung von Dialysierflüssigkeit angeschlossen ist, der ein Mehrkammerbeutel ist, dessen Kammern (2A, 2B) verschiedene Komponenten zur Herstellung der Dialysierflüssigkeit enthalten, und das andere Ende (3B) der Schlauchleitung an einem Einlass (14A) der zweiten Kammer (14) der Austauscheinheit (11) angeschlossen ist, wobei der in die okkludierende Schlauchpumpe einzulegende Schlauchabschnitt (3C) in eine okkludierende Schlauchpumpe (17) der extrakorporalen Blutbehandlungsvorrichtung zur Förderung von Dialysierflüssigkeit eingelegt ist.

15. Vorrichtung nach Anspruch 13, **dadurch gekennzeichnet, dass** an das eine Ende (3A) der Schlauchleitung (3) ein Beutel (2) zur Bereitstellung von Substituat angeschlossen ist, der ein Mehrkammerbeutel ist, dessen Kammern (2A, 2B) verschiedene Komponenten zur Herstellung des Substituats enthalten, und das andere Ende (3B) der Schlauchleitung (3A) an der venösen und/oder arteriellen Blutleitung (15, 16) des extrakorporalen Blutkreislaufs angeschlossen ist, wobei der in die okkludierende Schlauchpumpe einzulegende Schlauchabschnitt in die Subtituatpumpe (18) der extrakorporalen Blutbehandlungsvorrichtung eingelegt ist.

16. Vorrichtung nach einem der Ansprüche 10 bis 15, **dadurch gekennzeichnet, dass** die Blutbehandlungsvorrichtung eine Einrichtung (24; 27, 21) zur Erkennung eines Störfalls aufgrund einer verringerten oder unterbrochenen Flüssigkeitsströmung durch die Einrichtung (6) zum Auffangen von Partikeln aufweist.

17. Vorrichtung nach Anspruch 16, **dadurch gekennzeichnet, dass** die Einrichtung (24; 27, 21) zur Erkennung eines Störfalls mit einer Alarmeinheit (26) zusammenwirkt, die bei einem Störfall einen optischen und/oder akustischen und/oder taktilen Alarm gibt.

18. Vorrichtung nach Anspruch 16 oder 17, **dadurch gekennzeichnet, dass** die Einrichtung (24, 27) zur Erkennung eines Störfalls eine Auswerteinheit (24) und einen Druckaufnehmer (27) aufweist, der den Druck in dem Schlauchabschnitt (3E) misst, der zwischen dem in die okkludierende Schlauchpumpe einzulegenden Schlauchabschnitt(3C) und der Einrichtung (6) zum Auffangen von Partikeln liegt, wobei die Auswerteinheit (24) derart ausgebildet ist, dass der mit dem Druckaufnehmer gemessene Druck mit einem vorgegebenen maximalen Überdruck verglichen wird, wobei bei Überschreiten des Überdrucks ein Störfall erkannt wird.

19. Vorrichtung nach Anspruch 16 oder 17, **dadurch gekennzeichnet, dass** die Einrichtung (24, 21) zur Erkennung eines Störfalls eine Auswerteinheit (24) und eine Einrichtung (21) zum Wiegen eines Beutels zur Aufnahme von Dialysierflüssigkeit, Substituat oder Peritonellösung aufweist, wobei die Auswerteinheit (24) derart ausgebildet ist, dass die Gewichtsabnahme des Beutels innerhalb eines vorgegebenen Zeitintervalls mit einem vorgegebenen Grenzwert verglichen wird, wobei bei Unterschreiten des vorgegebenen Grenzwerts ein Störfall erkannt wird.

## Claims

1. Tube set for a blood treatment apparatus, having a tube line (3) that has a tube section (3C) to be laid into an occluding tube pump of the blood treatment apparatus, **characterized in that** a device for capturing particles (6) is provided in a tube section (3D) downstream from the tube section to be laid into the occluding tube pump, wherein a device (8) for limiting the excess pressure is provided, which is configured in such a manner that when a predetermined maximal excess pressure is exceeded in a tube section that lies downstream from the tube section (3C) to be laid into the occluding tube pump and upstream from the device (6) for capturing particles, a fluid connection between a tube section (3G) that lies downstream from the tube section to be laid into the occluding tube pump and a tube section (3E) that lies upstream from the tube section to be laid into the occluding tube pump is produced, and when the pressure drops below the predetermined maximal excess pressure, the fluid connection is interrupted.

2. Tube set according to claim 1, **characterized in that** the device (8) for limiting the excess pressure has a biased kick-back valve (10) that is open in the direction of the fluid flow, from downstream from the tube section (3C) to be laid into the occluding tube pump to upstream from the tube section to be laid into the occluding tube pump, when the predetermined maximal pressure is exceeded, and is closed when the pressure drops below the predetermined maximal pressure.

3. Tube set according to claim 2, **characterized in that** a first tube piece (9B) branches off from the tube section (3G) that lies downstream from the tube section (3C) to be laid into the occluding tube pump, the end of which piece is connected with the one connector (10B) of the biased kick-back valve (10), and that a second tube piece (9A) branches off from the tube section (3E) that lies upstream from the tube section (3C) to be laid into the occluding tube pump, the end of which piece is connected with the other connector (10A) of the biased kick-back valve (10).

4. Tube set according to claim 1, **characterized in that** the device (8) for limiting the excess pressure has an electromagnetically or pneumatically activated valve (10') that can be controlled by a control unit that interacts with a device for recognizing a problem caused by elevated flow-through resistance through the device for capturing particles, wherein the control unit is configured in such a manner the valve is open when a problem is recognized, so that a connection is produced between the tube section that lies downstream from the tube section to be laid into the occluding tube pump and the tube section that lies upstream from the tube section to be laid into the occluding tube pump, and the valve is closed when no problem is recognized.

5. Tube set according to claim 4, **characterized in that** a first tube piece (9B) branches off from the tube section (3G) that lies downstream from the tube section (3C) to be laid into the occluding tube pump, the end of which piece is connected with the one connector (10B) of the electromagnetically, hydraulically, or pneumatically activated valve (10'), and that a second tube piece (9A) branches off from the tube section (3E) that lies upstream from the tube section (3C) to be laid into the occluding tube pump, the end of which piece is connected with the other connector (10A) of the electromagnetically or pneumatically activated valve (10').

6. Tube set according to one of claims 1 to 5, **characterized in that** the device for capturing particles is a particle filter (6) that has a pore size of 0.1 µm to 100 µm, preferably 0.2 µm to 50 µm, particularly preferably 0.2 µm to 20 µm.

7. Tube set according to one of claims 1 to 6, **characterized in that** the tube set (1) has a bag (7) for heating the fluid that flows through the tube line.

8. Arrangement having a bag (2) for making available dialysis fluid or substituate for extracorporeal blood treatment or a peritoneal solution for peritoneal dialysis, and a tube set according to one of claims 1 to 7.

9. Arrangement according to claim 8, **characterized in that** the bag (2) for making available dialysis fluid, substituate or peritoneal solution is a multi-chamber bag, the chambers (2A, 2B) of which are filled with different components for the production of the dialysis fluid, the substituate or the peritoneal solution.

10. Apparatus for blood treatment, using a tube set according to one of claims 1 to 7.

11. Apparatus for blood treatment according to claim 10, **characterized in that** the blood treatment apparatus is an apparatus for peritoneal dialysis, having a line (3) for supplying a peritoneal solution and a line (29) for discharge of the peritoneal solution, and that the tube line (3) of the tube set (1) according to one of claims 1 to 7 is the line for supplying the peritoneal solution, wherein the tube line (3) of the tube set (1) is laid into an occluding tube pump (31) of the apparatus for peritoneal dialysis for conveying the peritoneal solution.

12. Apparatus for blood treatment according to claim 11, **characterized in that** a bag (2) for making peritoneal solution available is connected with one end (3A) of the tube line (3) of the tube set (1), which bag is a multi-chamber bag, the chambers (2A, 2B) of which contain different components for the production of the peritoneal solution.

13. Apparatus for blood treatment according to claim 10, **characterized in that** the blood treatment apparatus is an apparatus for extracorporeal blood treatment, having an exchange unit (11) that has a first chamber (13) that is part of an extracorporeal blood circuit (I), and has a second chamber (14) that is part of a fluid system (II), wherein the extracorporeal blood circuit (I) comprises an arterial blood line (15) that leads to an inlet (13A) of the first chamber (13) of the exchange unit (11) and a venous blood line (16) that exits from an outlet (3B) of the first chamber (13) of the exchange unit (11).

14. Apparatus according to claim 13, **characterized in that** a bag (2) for making dialysis fluid available is connected with the one end (3A) of the tube line (3) of the tube set (1), which bag is a multi-chamber bag, the chambers (2A, 2B) of which contain different components for the production of the dialysis fluid, and the other end (3B) of the tube line is connected with an inlet (14A) of the second chamber (14) of the exchange unit (11), wherein the tube section (3C) to be laid into the occluding tube pump is laid into an occluding tube pump (17) of the extracorporeal blood treatment apparatus for conveying dialysis fluid.

15. Apparatus according to claim 13, **characterized in that** a bag (2) for making substituate available is connected with the one end (3A) of the tube line (3), which bag is a multi-chamber bag, the chambers (2A, 2B) of which contain different components for the production of the substituate, and the other end (3B) of the tube line (3A [sic - should be 3]) is connected with the venous and/or arterial blood line (15, 16) of the extracorporeal blood circuit, wherein the tube section to be laid into the occluding tube pump is laid into the substituate pump (18) of the extracorporeal blood treatment apparatus.

16. Apparatus according to one of claims 10 to 15, **characterized in that** the blood treatment apparatus has a device (24; 27, 21) for recognizing a problem caused by reduced or interrupted fluid flow through the apparatus (6) for capturing particles.

17. Apparatus according to claim 16, **characterized in that** the device (24; 27, 21) for recognizing a problem interacts with an alarm unit (26) that gives off an optical and/or acoustic and/or tactile alarm in the event of a problem.

18. Apparatus according to claim 16 or 17, **characterized in that** the device (24, 27) for recognizing a problem has an evaluation unit (24) and a pressure sensor (27) that measures the pressure in the tube section (3E) that lies between the tube section (3C) to be laid into the occluding tube pump and the device (6) for capturing particles, wherein the evaluation unit (24) is configured in such a manner that the pressure measured with the pressure sensor is compared with a predetermined maximal excess pressure, wherein a problem is recognized when this excess pressure is exceeded.

19. Apparatus according to claim 16 or 17, **characterized in that** the device (24, 21) for recognizing a problem has an evaluation unit (24) and a device (21) for weighing a bag for accommodating dialysis fluid, substituate or peritoneal solution, wherein the evaluation unit (24) is configured in such a manner that the weight decrease of the bag within a predetermined time interval is compared with a predetermined limit value, wherein a problem is recognized when the value drops below the predetermined limit value.

## Revendications

1. Ensemble de conduite flexible pour un dispositif de traitement du sang avec une conduite flexible 3 qui comporte un segment de conduite flexible (3C) à placer dans une pompe péristaltique occlusive du dispositif de traitement du sang, **caractérisé en ce que**, dans un segment de conduite flexible (3D) en aval du segment de conduite flexible à placer dans la pompe péristaltique occlusive est prévu un système de capture de particules (6), un système (8) étant prévu pour limiter la surpression, qui est conçu de telle sorte que lors du dépassement d'une surpression maximale prédéterminée dans un segment de conduite flexible qui se trouve en aval du segment de conduite flexible (3C) à placer dans la pompe péristaltique occlusive et en amont du système (6) de capture de particules, est produite une liaison de fluide entre un segment de conduite flexible (3G) qui se trouve en aval du segment de conduite flexible à placer dans la pompe péristaltique occlusive, et un segment de conduite flexible (3E) qui se trouve en amont du segment de conduite flexible à placer dans la pompe péristaltique occlusive et lors du dépassement de la surpression maximale prédéterminée, la liaison de fluide est interrompue.

2. Ensemble de conduite flexible selon la revendication 1, **caractérisé en ce que** le système (8) pour limiter la surpression présente un clapet anti-retour (10) précontraint qui est ouvert lors du dépassement de la surpression maximale prédéterminée dans la direction de l'écoulement de liquide de l'aval du segment de conduite flexible (3C) à placer dans la pompe péristaltique occlusive à l'amont du segment de conduite flexible à placer dans la pompe péristaltique occlusive et est fermé lorsque la valeur est inférieure à la surpression maximale prédéterminée.

3. Ensemble de conduite flexible selon la revendication 2, **caractérisé en ce que**, à partir du segment de conduite flexible (3G) qui se trouve en aval du segment de conduite flexible (3C) à placer dans la pompe péristaltique occlusive, un premier morceau de conduite flexible (9B) se ramifie, dont une extrémité est reliée à un raccord (10B) du clapet anti-retour précontraint (10) et **en ce que**, à partir du segment de conduite flexible (3E) qui se trouve en amont du segment de conduite flexible (3C) à placer dans la pompe péristaltique, un deuxième morceau de conduite flexible (9A) se ramifie, dont une extrémité est reliée à l'autre raccord (10A) du clapet anti-retour précontraint (10).

4. Ensemble de conduite flexible selon la revendication 1, **caractérisé en ce que** le système (8) pour limiter la surpression présente une soupape (10') pouvant être actionnée de façon électromagnétique ou pneumatique, qui peut être commandée par une unité de commande qui coopère avec un système d'identification d'un incident en raison d'un accroissement de la résistance du débit dans le système de capture de particules, l'unité de commande étant conçue de telle sorte que la soupape soit ouverte lorsqu'un incident est identifié, de sorte qu'une liaison entre le segment de conduite flexible qui se trouve en aval du segment de conduite flexible à placer dans la pompe péristaltique occlusive et le segment de conduite flexible qui se trouve en amont du segment de conduite flexible à placer dans la pompe péristaltique occlusive soit produite et la soupape est fermée lorsqu'aucun incident n'est identifié.

5. Ensemble de conduite flexible selon la revendication 4, **caractérisé en ce que**, à partir du segment de conduite flexible (3G) qui se trouve en aval du segment de conduite (3C) flexible à placer dans la pompe péristaltique occlusive, un premier segment de conduite flexible (9B) se ramifie, dont une extrémité est reliée à un raccord (10B) de la soupape (10') pouvant être actionnée de façon électromagnétique, hydraulique ou pneumatique et **en ce que**, à partir du segment de conduite flexible (3E) qui se trouve en amont du segment de conduite (3C) flexible à placer dans la pompe péristaltique occlusive, un deuxième segment de conduite flexible (9A) se ramifie, dont une extrémité est reliée à l'autre raccord (10A) de la soupape (10') pouvant être actionnée de façon électromagnétique ou pneumatique.

6. Ensemble de conduite flexible selon l'une des revendications 1 à 5, **caractérisé en ce que** le système pour la capture de particules est un filtre à particules (6) qui présente une taille de pore de 0,1 µm à 100 µm, de préférence, de 0,2 µm à 50 µm, de manière particulièrement préférée, de 0,2 µm à 20 µm.

7. Ensemble de conduite flexible selon l'une des revendications 1 à 6, **caractérisé en ce que** l'ensemble de conduite flexible (1) présente une poche (7) pour le chauffage du liquide s'écoulant dans la conduite.

8. Agencement comportant une poche (2) pour la mise à disposition du liquide de dialyse ou d'un substitut pour un traitement extracorporel du sang ou d'une solution péritonéale pour une dialyse péritonéale et un ensemble de conduite flexible (1) selon l'une des revendications 1 à 7.

9. Agencement selon la revendication 8, **caractérisé en ce que** la poche (2) pour la mise à disposition du liquide de dialyse, du substitut ou de la solution péritonéale est une poche à plusieurs chambres dont les chambres (2A, 2B) sont remplies de différents composants pour la production du liquide de dialyse, du substitut ou de la solution péritonéale.

10. Dispositif pour le traitement du sang comportant un ensemble de conduite flexible selon l'une des revendications 1 à 7.

11. Dispositif pour le traitement du sang selon la revendication 10, **caractérisé en ce que** le dispositif pour le traitement du sang est un dispositif de dialyse péritonéale comportant une conduite (3) pour acheminer une solution péritonéale et une conduite (29) pour évacuer la solution péritonéale, et **en ce que** la conduite flexible (3) de l'ensemble de conduite flexible (1) selon l'une des revendications 1 à 7 est la conduite pour acheminer la solution péritonéale, la conduite flexible (3) de l'ensemble de conduite flexible (1) étant placée dans une pompe péristaltique occlusive (31) du dispositif de dialyse péritonéale pour l'acheminement de la solution péritonéale.

12. Dispositif pour le traitement du sang selon la revendication 11, **caractérisé en ce que**, à une extrémité (3A) de la conduite flexible (3) de l'ensemble de conduite flexible (1) est raccordée une poche (2) pour la mise à disposition de la solution péritonéale, qui est une poche à plusieurs chambres dont les chambres (2A, 2B) contiennent différents composants pour la production de la solution péritonéale.

13. Dispositif pour le traitement du sang selon la revendication 10, **caractérisé en ce que** le dispositif de traitement du sang est un dispositif pour le traitement extracorporel du sang comportant une unité d'échange (11) qui présente une première chambre (13) qui fait partie d'un circuit sanguin extracorporel (1) et une deuxième chambre (14) qui fait partie d'un système de liquide (11), le circuit sanguin extracorporel (1) comprenant une conduite de sang artérielle (15) conduisant jusqu'à une entrée (13A) de la première chambre (13) de l'unité d'échange (11) et une conduite de sang veineuse (16) partant d'une sortie (13B) de la première chambre (13) de l'unité d'échange (11).

14. Dispositif selon la revendication 13, **caractérisé en ce que**, à une extrémité (3A) de la conduite flexible (3) de l'ensemble de conduite flexible (1) est raccordée une poche (2) pour la mise à disposition du liquide de dialyse qui est une chambre à plusieurs poches dont les chambres (2A, 2B) contiennent différents composants pour la production du liquide de dialyse et l'autre extrémité (3B) de la conduite flexible est raccordée à une entrée (14A) de la deuxième chambre (14) de l'unité d'échange (11), le segment de conduite flexible (3C) à placer dans la pompe péristaltique occlusive étant placé dans une pompe péristaltique occlusive (17) du dispositif de traitement extracorporel du sang pour l'acheminement du liquide de dialyse.

15. Dispositif selon la revendication 13, **caractérisé en ce que**, à une extrémité (3A) de la conduite flexible (3) est raccordée une poche (2) pour la mise à disposition d'un substitut, qui est une chambre à plusieurs poches dont les chambres (2A, 2B) contiennent différents composants pour la production du substitut et l'autre extrémité (3B) de la conduite flexible (3A) est raccordée à la conduite de sang veineuse et/ou artérielle (15, 16) du circuit extracorporel du sang, le segment de conduite flexible à placer dans la pompe péristaltique occlusive étant placé dans la pompe de substitut (18) du dispositif de traitement extracorporel du sang.

16. Dispositif selon l'une des revendications 10 à 15, **caractérisé en ce que** le dispositif de traitement du sang présente un système (24 ; 27, 21) pour l'identification d'un incident en raison d'une réduction ou d'une interruption de l'écoulement de liquide à travers le système (6) de capture de particules.

17. Dispositif selon la revendication 16, **caractérisé en ce que** le système (24 ; 27, 21) d'identification d'un incident coopère avec une unité d'alarme (26) qui produit une alarme optique et/ou acoustique et/ou tactile en cas d'incident.

18. Dispositif selon la revendication 16 ou 17, **caractérisé en ce que** le système (24, 27) d'identification d'un incident présente une unité d'analyse (24) et un capteur de pression (27) qui mesure la pression dans le segment de conduite flexible (3E) qui se trouve entre le segment de conduite flexible (3C) à placer dans la pompe péristaltique occlusive et le système (6) de captures de particules, l'unité d'analyse (24) étant conçue de telle sorte que la pression mesurée avec le capteur de pression soit comparée à une surpression maximale prédéterminée, un incident étant identifié en cas de dépassement de la surpression.

19. Dispositif selon la revendication 16 ou 17, **caractérisé en ce que** le système (24, 21) d'identification d'un incident présente une unité d'analyse (24) et un système (21) de pesée d'une poche pour recevoir un liquide de dialyse, un substitut ou une solution péritonéale, l'unité d'analyse (24) étant conçue de telle sorte que la perte de poids de la poche dans un intervalle temporel prédéterminé est comparée à une valeur seuil prédéterminée, un incident étant identifié lorsque la valeur est inférieure à la valeur seuil prédéterminée.
